# EUROPEAN PATENT APPLICATION

(11) **EP 1 994 884 A1**
(43) Date of publication of application: **26.11.2008**
(21) Application number: 07738119.2
(22) Date of filing: 09.03.2007
(51) Int. Cl.: A61B 5/151

(54) **LANCET DEVICE**

(30) Priority: 10.03.2006 JP 2006065789
(71) Applicant: Arkray, Inc., Kyoto-shi, Kyoto 601-8045 (JP)
(72) Inventor: FUKUZAWA, Masahiro, Kyoto-shi, Kyoto 601-8045 (JP)
(74) Representative: Kastel, Stefan
(86) International application number: PCT/JP2007/054634
(87) International publication number: WO 2007/105617

(57) **Abstract**

At the lancet device (10), a structure is used as a shot prevention mechanism for preventing re-shooting using a used lancet (20), that inhibits movement to a shot-ready completion state where a puncture body (23) is held at different positions within a casing (22) before and after use. The holding position of the puncture body (23) within the casing (22) before and after use being different is then utilized. When the used lancet (20) is fitted to a main body (30), a stopper plate (38) does not operate, and a setting release button (37) remains in a pressed down state and is not pushed up.

## Description

### TECHNICAL FIELD

The present invention relates to a lancet device used for producing a puncture wound on the skin when fluid or the like is obtained through the skin.

### BACKGROUND ART

With the increase in diabetes patients in recent years, there has been an increase in diabetes patients measuring their own blood sugar levels at home or the like and managing changes in their blood sugar levels themselves. In light of this situation, lancet devices (puncture devices) equipped with puncture needles capable of easily creating a wound in a fingertip or the like and collecting the required blood for the measurements have been provided when blood is collected for measuring the blood sugar level.
A puncture needle is mounted at the tip of the lancet device. The puncture needle is shot by means of a spring force while the tip of the lancet device is put on the tip of a finger or the like, and is protruded from the tip of the lancet device so that the protruded length corresponds to a few millimeters to 2.0 millimeters. With the above operation, the tip of a finger or the like is cut by the puncture needle and blood bleeding from the puncture wound is obtained. The obtained blood is then dropped on a sensor portion or the like of a glucose meter. It is therefore possible to measure the blood sugar level.
As described above, this type of lancet device includes a puncture needle for producing a puncture wound on the tip of a finger or the like of a patient. For example, there has been a risk that especially older people with poor eyesight or the like may get hurt as a result of mishandling the puncture needle.
In view of this, a so-called safety lancet device has been proposed. The safety lancet device employs a configuration where a puncture needle is not exposed out of the main body if it is not needed.
For example, in patent document 1, it is ensured that the puncture needle does not protrude from the casing during normal use to ensure safety by using two coil springs and stipulating coefficients of elasticity for the springs.

### <Patent Document 1>

Japanese Patent Application Publication No. 2000-245717 (disclosed on September 12, 2000).

### DISCLOSURE OF THE INVENTION

### PROBLEMS THE INVENTIONS TO SOLVE

However, the conventional lancet device described above has the following problems.
In the lancet device disclosed in the publication described above, fixing of the lancet within the case after use is insufficient. It is therefore feared that the puncture needle will become unstuck and the tip of the needle will protrude from the case if the case is shaken after use. When the lancet is erroneously installed after use, the installation can take place without any problem. This means that re-puncturing is possible using a used puncture needle. There is therefore the fear that another person may become infected with an infectious disease and the like as a result of this secondary usage. It is therefore difficult to reliably ensure a high degree of safety after use in the configuration for a lancet device of the related art.
The present invention therefore provides a lancet device capable of ensuring safety after use with a simple structure.

### MEANS TO SOLVE THE PROBLEMS

A lancet device of a first aspect of the invention includes a lancet, a main body, an biasing member, and a shot-ready movement portion. The lancet includes a puncture body with a puncture needle at a tip, and a lancet case housing the puncture body within so as to be moveable in a puncture direction, with a holding position for the puncture body within the lancet case before puncturing and a holding position for the puncture body within the lancet case after removal once from the main body after puncturing being different. The main body includes an opening portion the lancet is inserted into, and a lancet holder for holding the lancet inserted from the opening portion. The biasing member is provided within the main body, and provides a biasing force for causing the puncture needle to protrude towards the front end side with the whole of lancet holder. The shot-ready movement portion is provided within the main body, and moves the lancet to a shot-ready state as a result of the biasing member only when the lancet is installed before puncturing.
In the lancet device including the lancet fitted to the main body, a holding position for the puncture body within the lancet case is different before puncturing and after being removed after puncturing one time. The lancet device includes the shot-ready movement portion capable of moving the lancet to a shot-ready state only when the puncture body within the lancet case is in a holding position before puncturing. In other words, the configuration is such that the shot-ready movement portion cannot cause movement to a shot-ready state when the lancet is removed from the main body and re-installed at the main body after puncturing. Further, the "puncture direction" refers to the direction of the puncture body moving in a direction to the front and to the rear within the lancet device when puncturing.
Here, the holding position for the puncture body within the lancet case before puncturing and the holding position for the puncture body within the lancet case after displacement from the main body after puncturing being different includes the case where, for example, the holding position is changed by holding the puncture body at a forward position of the lancet case before puncturing, and holding the puncture body in a state where movement is not possible at a retracted position within the lancet case when the lancet is distanced from the main body after puncturing. Further, the shot-ready movement portion includes a stopper plate etc., for example, which pushes the shot button upwards when the lancet is installed and makes the lancet device to move to a shot-ready state by pressing down the shot button.
As a result, when the lancet including the puncture body before use is installed at the main body, it is possible for the lancet to be moved smoothly to a shot-ready state. On the other hand, the holding position of the puncture body within the lancet case is different before puncturing when a lancet including a used puncture needle is erroneously reinstalled at the main body after a lancet including a puncture needle that has been used once is removed once from the main body. Movement of the lancet to a shot-ready state is then prohibited and use is not possible.
As a result, it is then possible to avoid the danger of an infectious disease occurring via a puncture needle by reliably prohibiting erroneous re-puncturing using lancets including puncture needles that have already been used. It is therefore possible to provide a safer lancet device.
A lancet device of a second aspect of the invention is the lancet device according to the first aspect of the invention, wherein the lancet includes a puncture body, a lancet case, and a fitting portion. The puncture body includes a linking portion which is formed at an end portion on an opposite side to the puncture needle and is linked with the main body. The lancet case includes a tubular portion housing the puncture body in a state where forward and reverse movement is possible in the puncture direction, and an opening formed at an end portion of the protruding side of the puncture needle. The fitting portion holds the puncture body within the lancet case in a state where forward and revere movement in the puncture direction is not possible when the linking portion of the puncture body is removed from of the main body.
Here, when the lancet case is removed from the main body after a puncture wound is formed, the puncture body is forcibly held in a state where movement in the puncture direction is not possible by the fitting portion.
Here, a combination of a concave portion or a convex portion formed at an outer periphery of the puncture body and a convex portion or concave portion formed at the surface of the inside of a tubular portion of the lancet case housing the puncture body can be considered as the fitting portion. When the lancet of the present invention is removed from the main body, the concave portion or the convex portion formed at the puncture body fits with the convex portion or the concave portion formed at the surface of the inside of the tubular portion of the lancet case and the lancet is forcibly held within the lancet case in a state where movement of the puncture body in the puncture direction is not possible.
As a result, it is possible to prevent the puncture needle from protruding from the tip of the puncture body without covering the tip of the puncture needle with a cap when removing and discarding the puncture body from the main body after forming the puncture wound. Therefore, even with a simple structure, it is possible to prevent a user from being injured by a puncture needle erroneously protruding from the tip of the puncture body when the lancet is discarded and it is possible to prevent the danger of infection with an infectious disease due to fluid etc. affixed to a used puncture needle.
Further, even when an already used lancet is erroneously fitted again to the main body, in case the lancet is removed once from the main body, the puncture body is forcibly held in a state where movement in the puncture direction is not possible by the fitting portion. It is therefore possible to prohibit the puncture needle from being shot. As a result, the danger of infection due to the reuse of blood-stained puncture needles is reduced and it is possible to provide a safer lancet.
A lancet device of a third aspect of the invention is the lancet device according to the first or second aspect of the invention, the shot-ready movement portion moves the lancet to a shot-ready state in accordance with movement of the lancet holder in the puncture direction. The main body is then further provided with a movement regulating portion that regulates movement of the lancet holder holding the lancet in the puncture direction when the lancet after puncturing is installed.
Here, when attempts are made to install a lancet removed once from the main body after puncturing with the intention of erroneous reuse, movement of the lancet holder holding the inserted lancet is regulated by the movement regulating portion formed within the main body so as not to allow movement to the back from a predetermined position in the puncture direction.
As a result, it is possible to reliably inhibit movement to a shot-ready state as a result of a shot button being pressed upwards by regulating movement of the lancet holder in the mechanism for moving to a shot-ready state by the shot-ready movement portion in accordance with movement of the lancet holder. As a result, with a simple structure, it is possible to prevent secondary use of an already used lancet and it is possible to provide a very safe lancet device.
A lancet device of a fourth aspect of the invention is the lancet device according to one of the first to third aspects of the invention, further comprising a shot button pressed when shooting the lancet. The shot-ready movement portion pushes the shot button that is normally pressed down upwards to make the lancet to a shot-ready state when the lancet is installed at the main body before puncturing.
Here, the shot button is pushed upwards and the lancet moves to a shot-ready state only when a lancet is installed before use (before puncturing).
As a result, the shot button remains in a pressed down state and does not pushed up even when it is intended to reinstall a lancet including a used puncture needle. As a result, it is possible to reliably prevent a used lancet from being reinstalled and being used again for puncturing.
A lancet device of a fifth aspect of the invention is the lancet device according to the fourth aspect of the invention, the shot-ready movement portion includes a stopper plate that enables the shot button to be pushed upwards to make the lancet to move to a shot-ready state in accordance with movement of the lancet case in the puncture direction during installation of the lancet.
Here, the stopper plate that moves to the rear in the puncture direction in accordance with movement in the puncture direction of the lancet case holding the puncture body and pushes the shot button upwards either directly or indirectly is used as a mechanism for pushing the shot button upwards to make the lancet to move a shot-ready state.
At the lancet included in the lancet device of the present invention, the position of the puncture body in the lancet case before use and after use is different.
It is therefore possible to move the stopper plate as a result of movement of the lancet case to different positions taking the puncture body as a reference before use and after use and it is possible to move the shot button to a shot-ready state. As a result, it is possible to reliably avoid re-puncturing using a used puncture needle even in cases where a lancet is erroneously re-installed to the main body after use because the shot button is not moved to a shot-ready state.

### BRIEF DESCRIPTION OF THE DRAWINGS

Fig 1 is a perspective view showing the appearance of a lancet device according to an embodiment of the present invention;
Fig. 2 is a transparent view showing a lancet installed to the main body of the lancet device of Fig. 1;
Fig. 3 is a cross-sectional view showing a structure for a lancet device main body and a lancet fitted to the main body of the lancet device of Fig. 1;
Fig. 4 is an enlarged view showing a structure for the lancet of Fig. 3;
Fig. 5(a) to Fig. 5(c) are cross-sectional views showing a flow during installation of the lancet to the main body before use;
Fig. 6(a) and (b) are cross-sectional views showing a flow during installation of the lancet to the main body before use;
Fig. 7(a) to (c) are cross-sectional views showing a flow during installation of the lancet to the main body after use;
Fig. 8(a) and (b) are cross-sectional views showing a flow during installation of the lancet to the main body after use; and
Fig. 9 is a side view showing a structure for an inner wall section of a housing constituting the outline of the main body of Fig. 3.

### DESCRIPTION OF THE REFERENCE NUMERALS

- 10: lancet device
- 20: lancet
- 21: puncture needle
- 22: casing (lancet case)
- 22a: cylindrical portion (tubular portion)
- 22b: pinching portion (fitting portion)
- 22c: groove
- 23: puncture body
- 23a: tapered portion
- 23b: flange portion
- 23c: groove (fitting portion)
- 23d: insertion portion (linking portion)
- 24: cap (cap portion)
- 24a: protruding portion (second fitting portion)
- 24b: lid member
- 24c: flange portion
- 30: main body (main body portion)
- 31: coil spring (biasing member)
- 32: lancet holder
- 32a: lancet insertion opening
- 32b: sleeve
- 32c: latch (shot-ready movement portion)
- 33: rotator
- 35: housing
- 35a: puncture hole (opening portion)
- 35b: opening
- 35c: rib (movement regulating portion)
- 35d: rib
- 36: removing portion
- 37: setting release button (shot button)
- 37a: protruding portion
- 38: stopper plate (shot-ready movement portion)
- 39: abutting stopping member
- x1, x2: distance

### BEST MODE FOR CARRYING OUT THE INVENTION

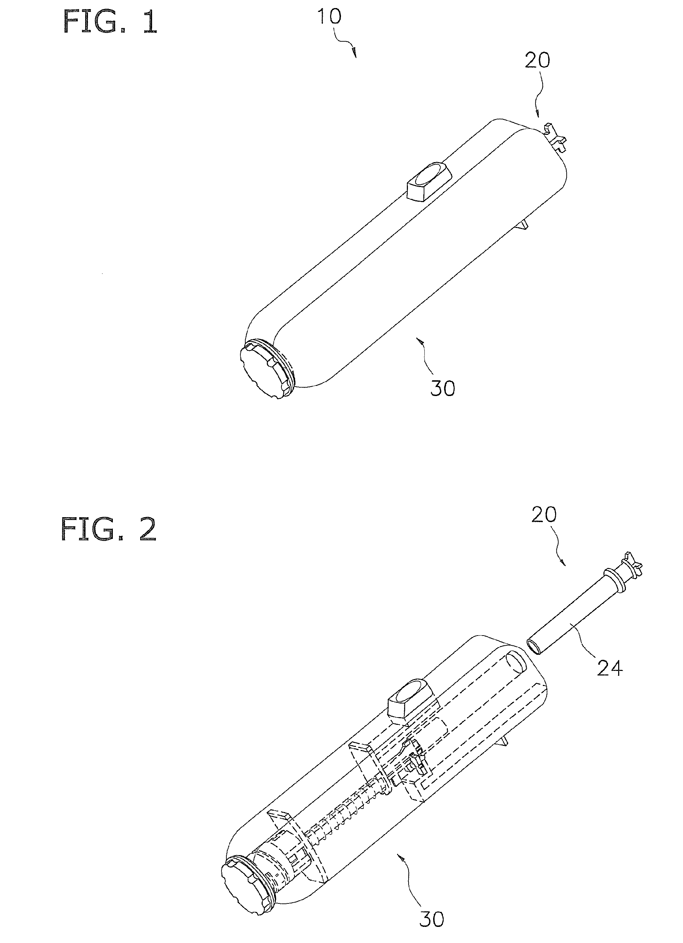

A lancet device in accordance with an embodiment of the present invention will be hereinafter explained with reference to Figs. 1 to 9.

### <ENTIRE CONFIGURATION OF LANCET DEVICE 10>

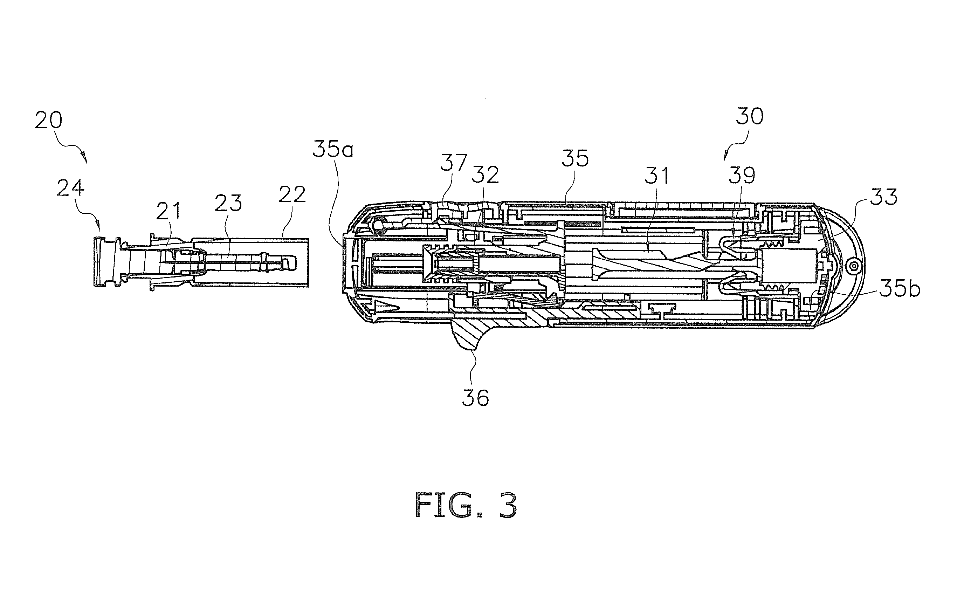

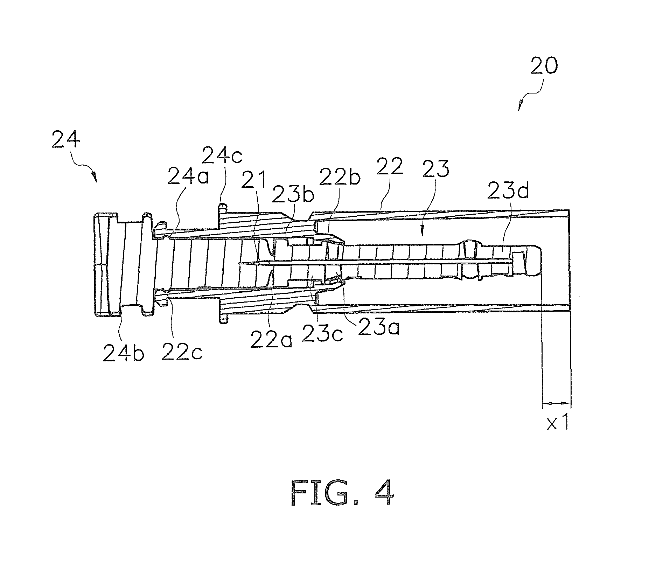

The lancet device 10 in accordance with an embodiment of the present invention is a device used for taking fluid from a diabetes patient when measurement of the blood sugar level or the like is performed for the patient. When the lancet device 10 is used, a puncture wound is formed on the skin by protruding a puncture needle 21 (refer to Fig. 4) from an opening formed on a tip of the lancet device 10 while the tip portion is in press-contact with the skin.
Specifically, as illustrated in Figs. 1 and 2, the lancet device 10 includes a lancet 20 and a main body (main body portion) 30.
The lancet 20 includes the stainless steel puncture needle 21 (refer to Fig. 4) inside for forming a puncture wound thereof, and as illustrated in Fig. 2, the lancet 20 is attached to the main body 30 from the front end side of the main body 30.
The main body 30 includes a coil spring 31 (refer to Fig. 3) that applies force to the puncture needle 21 (refer to Fig. 4) for making the puncture needle 21 protrude in a predetermined puncture direction and a return spring (not shown) that returns the puncture needle 21 projected by the coil spring 31 into the housing 35.
In the following explanation, "front end side" is used to refer to the front side in the puncture direction the tip of the puncture needle 21 of the lancet 20 described in the following protrudes in, and "rear end side" refers to the rear direction in the puncture direction on the opposite side.

### <CONFIGURATION OF LANCET 20>

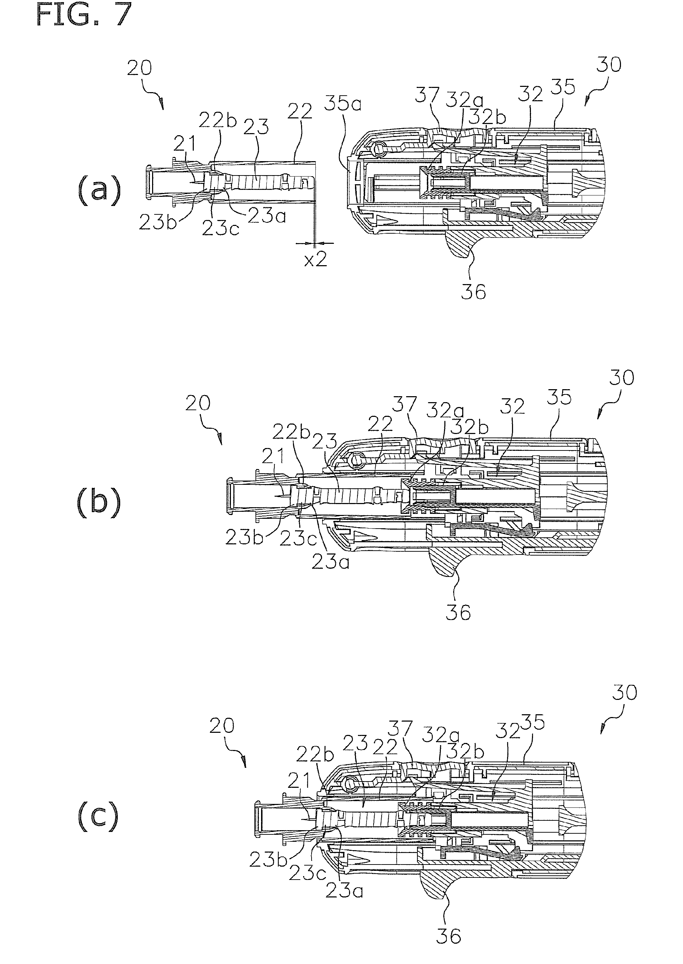

As illustrated in Fig. 4, the lancet 20 includes an approximately cylindrical-shaped casing (lancet case) 22, and a puncture body 23 as to be accommodated in the casing 22 so as to be allowed to move to the front and rear end sides of the puncture direction when the lancet device 10 is used. Note that Fig. 3 illustrates a cross-sectional view of the casing 22 in order to conveniently explain the interior configuration of the casing 22 having a substantially cylindrical-shape.
The puncture body 23 is made from resign and formed as a unit with the puncture needle 21 for forming the puncture wound in the skin. In addition, a tapered portion 23a, a flange portion 23b, a groove (fitting portion) 23c, and an insertion portion 23d are formed at a part of the puncture body 23 formed of resin. The tapered portion 23a, flange portion 23b and groove 23c, are formed in the tip side where the puncture needle 21 protrudes. The tapered portion 23a is a member that is formed to taper towards the rear end side, with a cross-section perpendicular to the puncture direction formed in an oval shape. The flange portion 23b is a disc-shaped member that is formed at the outmost of the front end side of the puncture body 23, and the puncture needle 21 protrudes from the center portion of the disc. The groove 23c is a concave portion formed to be interposed between the tapered portion 23a and the flange portion 23b. When the lancet 20 is removed from the main body 30 after the puncture body 23 is used, the puncture body 23 is moved to the rear end side relative to the casing 22, and a pinching portion (fitting portion) 22b of the casing 22 to be described fits into the groove 23c. The puncture body 23 can then be held within the casing 22. The insertion portion 23d is inserted into the lancet holder 32 of the main body 30 to be described later. At this time, it is possible to hold the puncture body 23 at the lancet holder 32 by moving the tip portion of the lancet holder 32 towards the inside and fastening the insertion portion 23d. Thus, the puncture body 23 along with the lancet holder 32 moves forward in the puncture direction by the elastic force of a coil spring 31 disposed at the rear end side of the lancet holder 32 in the main body 30.
In addition, as illustrated in Figs. 3 and 4, a cap 24 is attached to the puncture needle 21 so as to cover the tip thereof, and prevents the tip of the puncture needle 21 from being exposed outside before the lancet 20 is used. The cap 24 is integrally formed with the puncture needle 21 as is the case with the puncture body 23, and a portion of the cap 24 is connected to the flange portion 23b of the puncture body 23. Therefore, the part where the cap 24 and the flange portion 23b are connected may be separated by twisting and removing the cap 24 at the time of use, and the puncture needle 21 may be exposed inside the casing 22. In addition, the cap 24 includes a protruding portion 24a, a lid member 24b, and a flange portion 24c. The protruding portion 24a is a portion formed to protrude in a direction intersecting the puncture direction, and as illustrated in Fig. 4, is fitted with a groove 22c that is to be described and is formed in the end portion of the casing 22 on the front end side, when the cap 24 is mounted to the casing 22. In this configuration, it is possible to retain the puncture body 23 inside of the casing 22 before the lancet 20 is used. The lid member 24b functions as a lid for covering the outmost front end portion of the casing 22 before the lancet 20 is used. The flange portion 24c is a disc-shaped plate member formed on an upper portion of the cap 24. Blood etc. bleeding from a fingertip etc. at the time of puncturing is prevented from permeating to within the lancet device 10 from the puncture hole (opening portion) 3 5 a of the main body 30.
The casing 22 is a substantially cylindrical member, and houses the puncture body 23 inside from before use to disposal after use. As shown in Fig. 4, the casing 22 includes a cylindrical portion (tubular portion) 22a, a pinching portion (fitting portion) 22b, and a groove 22c. The cylindrical portion 22a is formed to have radius that is slightly larger than that of the tapered portion 23a and the flange portion 23b etc. of the puncture body 23. When the lancet 20 is used, the puncture body 23 moves in the front and rear end sides of the puncture direction. The pinching portion 22b is an elastic member that protrudes inwardly from the cylindrical portion 22a of the casing 22, and is formed in the vicinity of the center portion of the casing 22 in the longitudinal direction. When the lancet 20 is disposed of after use, the puncture body 23 is retracted to the rear end side, and the groove 23c of the puncture body 23 fits with the pinching portion 22b. Thus, protrusion of the puncture needle 21 from the opening at the front end side of the casing 22 after use is prevented, and safety may be assured after use. The groove 22c is a concave portion that is formed on the cylindrical portion 22a on the front end side of the casing 22. The protruding portion 24a of the cap 24 is engaged with the groove 22c before the lancet 20 is used. Therefore, it is possible to retain the puncture body 23 in the interior of the casing 22 so that the puncture body 23 is not allowed to move to the front and rear end sides in the puncture direction.
In the state before use, as shown in Fig. 4, the position of the puncture body 23 within the casing 22 is held comparatively to the tip side within the casing 22 on the one hand, while the position is held to the rear within the casing 22 as shown in Fig. 7(a) in the state where the lancet 20 is removed from the main body 30 once after use. Namely, when a distance x1 between the tip on the rear end side of the casing 22 and the tip on the rear end side of the puncture body 23 before use and a distance x2 between the tip on the rear end side of the casing 22 and the tip of the rear end side of the puncture body 23 after use are compared, then x1>x2. In this way, it is possible to reliably inhibit the lancet 20 from being reinstalled to the main body 30 and used for puncturing after use as described in the following by changing the position of the puncture body 23 within the casing 22 before and after use. The operation of each part when the lancet 20 is installed at the main body 30 before and after use is described later.

### <CONFIGURATION OF MAIN BODY 30>

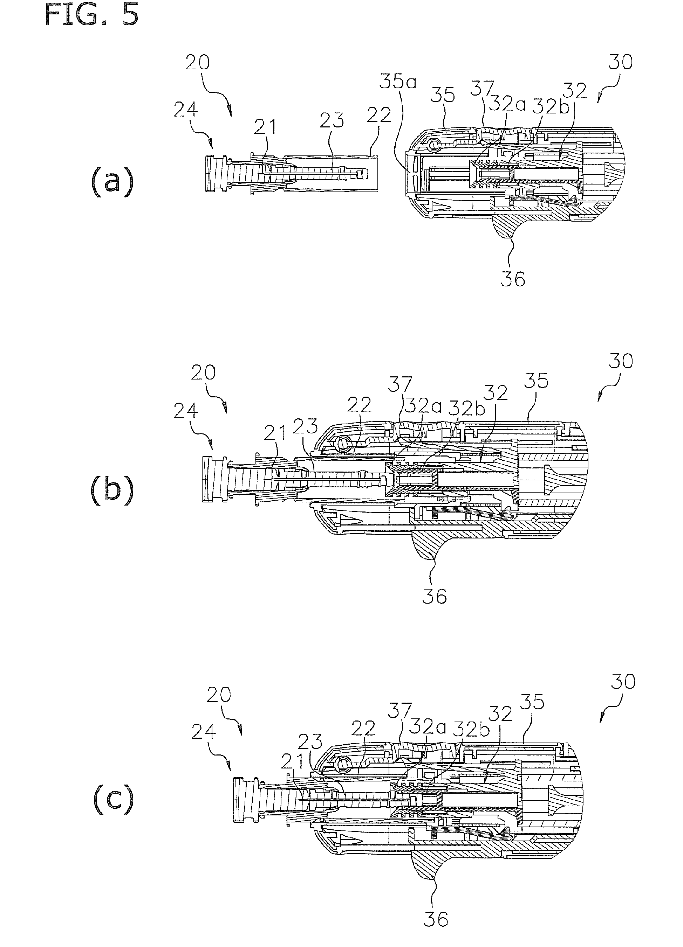

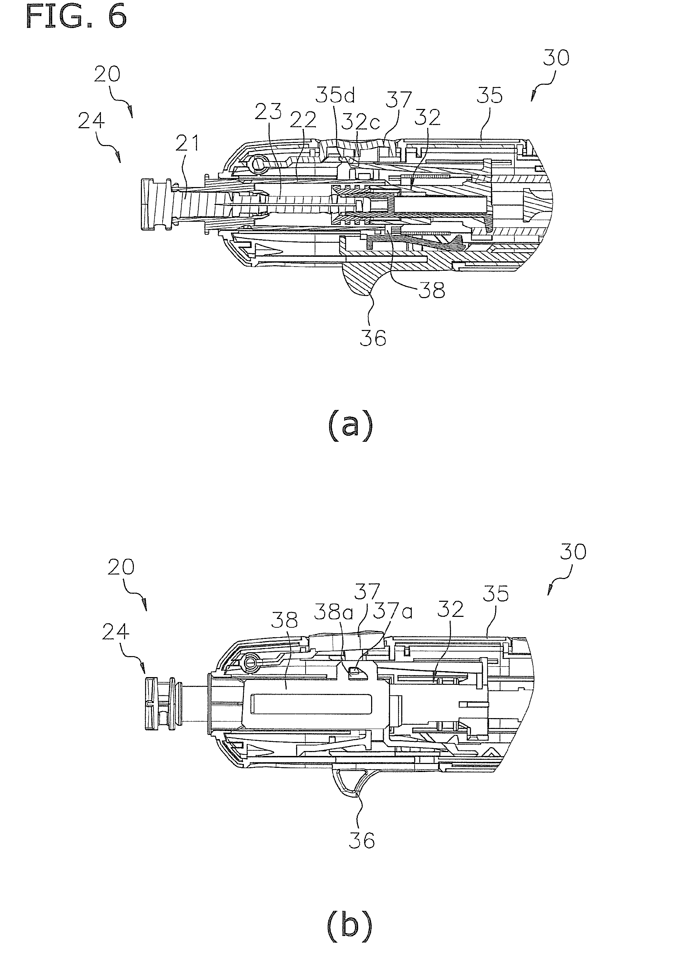

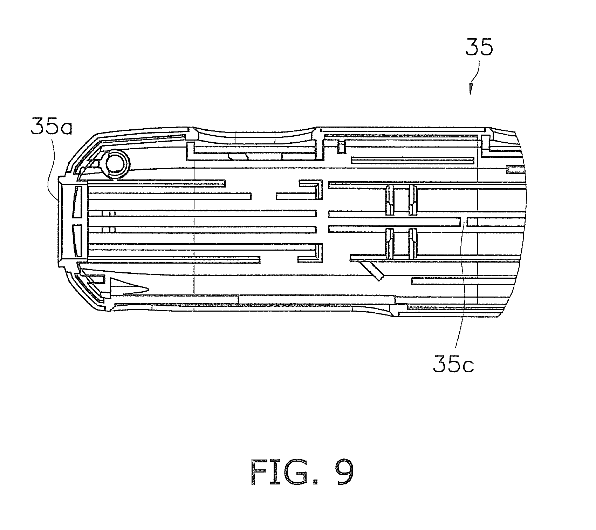

As illustrated in Fig. 3, the main body 30 includes the coil spring 31, the lancet holder 32, a rotator 33, the housing 35, a removing portion 36, a setting release button (shot button) 37, and the stopper plate (shot-ready movement portion) 38 (refer to Fig. 6(b)), and the above described lancet 20 is attached to the main body 30 from the front end side thereof (refer to Fig. 2).
The coil spring 31 is a member that applies force for moving the puncture body 23 of the lancet 20 forward in the puncture direction and is disposed at the rear end side of the lancet holder 32.
At the lancet holder 32, the rear end portion of the lancet 20 is held as a result of the portion (insertion portion 23d) of the rear end side of the lancet 20 inserted from the puncture hole 35a formed at the tip of the housing 35 being inserted while broadening as a result of pushing into the sleeve 32b (refer to Fig. 5(a) etc.) via the lancet insertion opening 32a (refer to Fig. 5(a) etc.). Further, the lancet holder 32 extends along the side surface thereof and has a latch portion (shot-ready movement portion) 32c (refer to Fig. 6(a)) that elastically deforms in a direction intersecting to the puncture direction. The latch portion 32c has a biasing force biasing upwards in the puncture direction and the setting release button 37 is pushed upwards by the biasing force into a shot-ready completion state (shot-possible state).
The rotator 33 rotates around the axial direction in the circumferential direction when the dial portion is rotated. Further, the rotator 33 includes a spiral-shaped rib that is formed on the inner surface of the cylindrical portion on the front end side of the dial portion. When the lancet holder 32 is biased towards the front end side by biasing force of a spring, the tip of the rear end side (not shown) of the lancet holder 32 abuts with part of the abutting stopping member 39 and movement in the puncture direction is regulated. As a result, it is possible to change the contact position of the convex portion and the abutting stopping member 39 by causing the rotator 33 to rotate. As a result, it is possible to adjust the position of the puncture body 23 in the puncture direction to the front and to the rear. It is also possible to adjust the extent of protrusion of the puncture needle 21 by rotating the rotator 33 before puncturing the skin and it is therefore possible to control puncture depth.
The housing 35 contains the coil spring 31 described above and the lancet holder 32 etc. built-in, and constitutes the outer wall of the lancet device 10. The housing 35 includes the puncture hole 35a at the tip of the front end side, the opening 35b at the tip of the rear end side, and the rib (movement regulating portion) 35c at the inner wall surface (refer to Fig. 9). The puncture hole 35a enables insertion of the lancet 20 and enables the tip of the needle of the puncture needle 21 to protrude when puncturing is performed. The opening 35b houses the rotator 33 and is formed in a circular shape matching the shape of the rotator 33. The rib 35c prohibits the lancet 20 from being reinstalled and used for puncturing after use as described in the following by regulating movement to the rear in the puncture direction with abutting of a part of the lancet holder 32, when the lancet 20 is reinstalled after use.
The removing portion 36 is exposed to a side opposed to the side which the setting release button 37 is exposed on the approximately rectangular housing 35. In addition, the removing portion 36 is disposed in the interior of the housing 35 so as to make contact with the tip of the rear end side of the casing 22. After completion of the puncture, the removing portion 36 is moved to the front end side. Only the casing 22 abutting with the removing portion 36 then moves forward towards the tip side. The pinching portion 22b of the casing 22 then fits with the groove 23c of the puncture body 23. When the removing portion 36 is moved forward, a part of the removing portion 36 is advanced to the holding portion of the lancet holder 32. Holding of the puncture body 23 (insertion portion 23d) at the lancet holder 32 is then released. It is then possible to remove the lancet 20 from the main body 30.
More specifically, in the stage before withdrawal of the lancet 20 from the main body 30, the puncture body 23 is moved to the rear end side relatively with respect to the casing 22 as a result of the casing 22 of the lancet 20 being pushed out more to the front end side than the puncture body 23. Within the lancet 20, the tapered portion 23a formed in the vicinity of a central part of the puncture body 23 moves while riding up on the pinching portion 22b formed at the cylindrical portion 22a of the casing 22. As a result, the vicinity of the pinching portion 22b formed at the cylindrical portion 22a of the casing 22 is elastically deformed.
The tapered portion 23a then moves to the rear end side. The puncture body 23 is then held within the casing 22 at the point where the pinching portion 22b fits with the groove 23c of the puncture body 23. At this time, it is therefore possible to move the puncture body 23 smoothly to the fitting position because the rear end side of the tapered portion 23a is thinner than the front end side thereof.
The setting release button 37 is exposed at the surface of the opposite side to the removing portion 36 at the substantially right-angled parallelepiped-shaped housing 35. A cocked state is therefore released at the same time as installing the lancet 20 by pressing down the setting release button 37 and puncturing can be carried out. The latter stage of movement to a state where a shot is possible at the setting release button 37 is described in the following stage.
The stopper plate 38 is disposed along a side of the lancet holder 32 within the housing 35. The presence or absence of movement of the setting release button 37 to a shot-ready state is then decided by changing the amount of movement in the puncture direction at the time of installation of the lancet 20 before use and at the time of re-installation of the lancet 20 after use.
A re-cocking lever provided normally in a typical lancet device is not provided in the lancet device 10 of the embodiment. It is therefore possible to prevent a used puncture needle from being re-cocked and shot after puncturing once by not providing a mechanism enabling re-cocking. It is therefore possible to reliably reduce the danger in the case of installation of used lancets.
"Flow from installation of unused lancet 20 to removal of lancet 20 after puncturing"
As shown in Fig 5(a), when the use of the lancet device 10 of the present embodiment begins, an unused, new lancet 20 is first inserted into the puncture opening 35a of the main body 30.
When the lancet 20 is then inserted deeply into the puncture opening 35a, as shown in Fig. 5(b), the insertion portion 23d formed at the tip of the rear end side of the puncture body 23 is inserted into the lancet insertion opening 32a of the tip of the lancet holder 32. When the lancet 20 is then inserted further to the back, as shown in Fig. 5(c), the rear end portion (the insertion portion 23d) of the puncture body 23 is inserted into the lancet holder 32 while the sleeve 32b spreads out to the outer side. The puncture body 23 is then held at the front end portion (lancet insertion opening 32a) of the lancet holder 32 so that the outer peripheral surface of the puncture body 23 is grasped by the sleeve 32b.
Next, when the lancet 20 is inserted as far as the back, as shown in Fig. 6(a), the rear end portion of the casing 22 abuts with part of the stopper plate 38 while the rear end portion (insertion portion 23d) of the puncture body 23 pushes the lancet holder 32 into the rear. At this time, the latch portion 32c of the lancet holder 32 is pushed down by the rib 35d formed at the inner wall surface of the housing 35.
Next, when the lancet 20 is inserted in as far as the back, the stopper plate 38 that the casing 22 abuts against is moved to the rear. The latch portion 32c of the lancet holder 32 then comes away from the rib 35d formed at the inner wall surface of the housing 35 and moves upwards. As a result, as shown in Fig. 6(b), the setting release button 37 is pushed upwards so that the coil spring 31 is cocked in the compression direction and the puncture needle 21 adopts a shot-ready state. As shown in Fig. 6(b), the setting release button 37 pushed upwards by the latch portion 32c is in a state where upward movement is regulated by catching on the protruding portion 37a at the opening 38a formed at a part of the stopper plate 38.
As a result, installation of the lancet 20 to the main body 30 is completed and movement towards the shot-ready state is possible.
Next, the cap 24 that is formed as a unit with the puncture body 23 is removed to expose the puncture needle 21. The cap 24 is connected partially to a surface of the flange portion 23b of the puncture body 23 on the front end side. Therefore, the cap 24 is removed when the connected portion is torsionally-sheared by rotating the cap 24. At this time, the pulling force to the front end side is applied for the cap 24 to be removed. Therefore, the pulling force to the front end side is also applied to the puncture body 23, a portion of which is connected to the cap 24. However, when the cap 24 is removed, the tip of the rear end side of the puncture body 23 is held by the lancet holder 32. The force of fitting of the puncture body 23 and the lancet holder 32 is then larger than the force required to detach the cap 24 from the puncture body 23. As a result, even if the cap 24 is removed from the casing 22, the puncture body 23 is not pulled out together with the cap 24 but rather is held within the casing 22.
Next, when the setting release button 37 is pressed with the puncture opening 35a in a state of contact with the skin to be punctured and the setting is released, the puncture needle 21 protrudes a predetermined amount from the puncture hole 35a formed in the very tip side of the main body 30 and puncturing can be performed. Then, the puncture needle 21 is brought back to the casing 22 again by means of the spring force of the return spring (not illustrated in the figures) immediately after a puncture is performed.
After the puncture is completed, the lancet 20 is removed from the main body 30 and is then disposed. Removal of the lancet 20 from the main body 30 is done using the removing portion 36. In other words, the hold on the puncture body 23 by the lancet holder 32 is released by moving the removing portion 36 towards the front end side, and the lancet 20 may be removed from the puncture hole 35a. Specifically, when the removing portion 36 is moved to the front end side, only the casing 22 is first moved to the front end side. Accordingly, the puncture body 23 that is being retained in the lancet holder 32 is moved to the rear end side relative to the casing 22. Here, the tapered portion 23a that is formed in the vicinity of the center portion of the puncture body 23 is moved while pressing and enlarging the pinching portion 22b formed on the cylindrical portion 22a of the casing 22 to the outside. The pinching portion 22b of the casing 22 is therefore engaged with the groove 23c of the puncture body 23. This engaged state is tightly formed, and therefore this makes it possible to prevent the tip of the puncture needle 21 from protruding from the front end side of the casing 22 after the lancet 20 is removed from the main body 30. With this configuration, the puncture needle 21 is prevented from protruding with the cap 24 before the lancet 20 is used, and the puncture body 23 is retained in the casing 22 by means of the engaged state with large engagement force after the lancet 20 is used. Therefore, it is possible to avoid the danger of the puncture needle 21 projecting from the tip of the casing 22 before and after the lancet 20 is used.
After the pinching portion 22b of the casing 22 is fitted in the groove 23c of the puncture body 23, the sleeve 32b of the lancet holder 32 moves relatively in the tip direction with respect to the lancet insertion opening 32a. The holding of the puncture body 23 is then released by the lancet holder 32 as a result of the force encompassing the insertion portion 23d of the puncture body 23 weakening.
As shown by the above procedure, in the lancet device 10 of the embodiment, the lancet 20 is removed from the main body 30 after use and is disposed. However, the puncture body 23 is tightly retained in the casing 22 by means of the engagement force in the removed lancet 20. Therefore, it is possible to reliably prevent such cases that a user is injured by the tip of the puncture needle 21 protruded from the casing 22 and that a user is infected by a disease through a fluid or the like attached to the puncture needle 21. Further, the lancet 20 removed once from the main body 30 holds the puncture body 23 within the casing 22 at a different position to before use. It is therefore possible to make it difficult to install the lancet 20 in the main body 30 again for reuse after use compared to the lancet of the related art.

### <Shot-inhibit mechanism at the time of reinstallation of a used lancet 20>

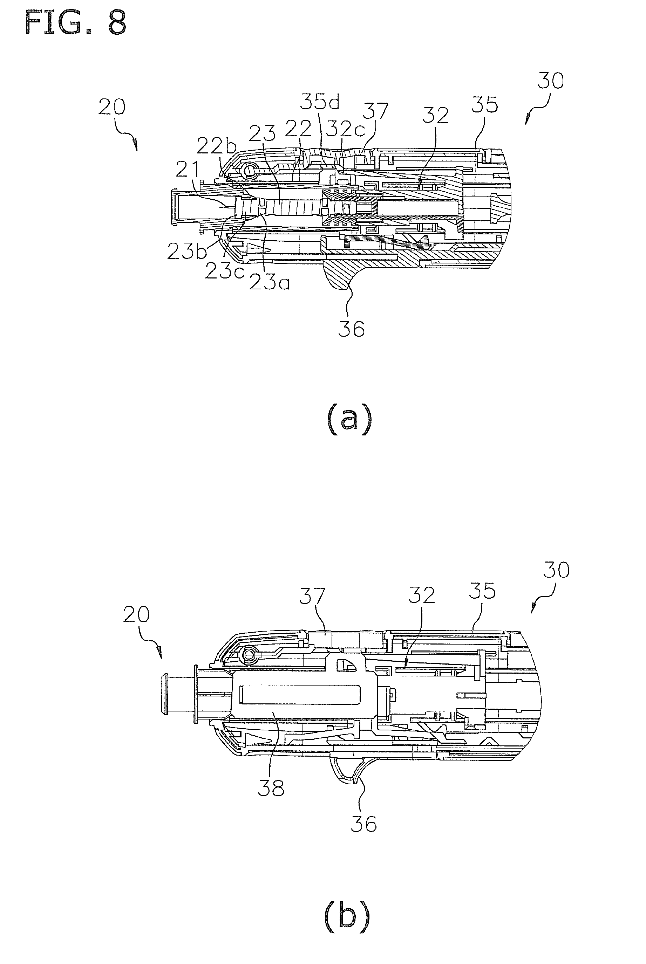

As described above, with the lancet device 10 of the embodiment, reuse of the lancet 20 after use is difficult because the puncture body 23 is firmly held within the casing 22. However, there is also the fear that the lancet 20 will be forcibly installed at the main body 30 for reuse after being used. The following mechanism is therefore further provided at the lancet device 10 of the embodiment as a mechanism for reliably inhibiting reuse of a used lancet 20.
When installation of a used lancet 20 (in other words, a lancet 20 that has been removed from the main body 30 after puncturing once) to the main body 30 again is attempted, first, the position of the puncture body 23 within the casing 22 is different compared to before use of the lancet 20.
Specifically, before use, the used lancet 20 is held to the rear end side within the casing 22 as shown in Fig. 7(a) whereas the puncture body 23 is held comparatively to the front end side within the casing 22, as shown in Fig. 4.
As shown in Fig. 7(a), when the used lancet 20 is then inserted into the puncture opening 35a of the main body 30, as with the time of insertion of the lancet 20 before use, as shown in Fig. 7(b), the insertion portion 23d formed at the tip of the rear end side of the puncture body 23 is inserted into the lancet insertion opening 32a of the tip of the lancet holder 32. When the lancet 20 is then inserted further to the back, as shown in Fig. 7(c), the rear end portion (insertion portion 23d) of the puncture body 23 enters the lancet holder 32 while the sleeve 32b spreads out to the outer side. The puncture body 23 is then held at the front end portion (lancet insertion opening 32a) of the lancet holder 32 so that the outer peripheral surface of the puncture body 23 is grasped by the sleeve 32b.
When the lancet 20 is inserted further to the back, as shown in Fig. 8(a), before the rear end portion of the casing 22 abuts with the stopper plate 38, a part of the lancet holder 32 makes contact with the rib 35c (refer to Fig. 9) formed at a movement boundary point at an inner wall surface of the housing 35 and the lancet 20 cannot be inserted any further to the back. Before and after use of the lancet 20, the cause of the difference in movement within the main body 30 is the difference in the holding position for the puncture body 23 with respect to the casing 22 before installation. In the lancet 20 after use, the puncture body 23 is held further towards the rear end with respect to the casing 22. The timing with which the rear end of the puncture body 23 comes into contact with the lancet holder 32 is therefore faster compared to the lancet 20 before use.
As a result, as shown in Fig. 8(b), the lancet holder 32 reaches a predetermined limit position and the lancet 20 cannot be inserted any further back. The casing 22 then abuts with the stopper plate 38 and the latch portion 32c of the lancet holder 32 is opened up from the rib 35d formed at the inner wall surface of the housing 35 by the stopper plate 38 so as to move upwards. The setting release button 37 is then pushed upwards and the achievement of a shot-ready completion state can be inhibited.
As described above, in the lancet device 10 of the embodiment, the setting release button 37 for enabling puncturing is pushed up so as to prohibit to move the used lancet 20 to a shot-ready completion state even in cases where the used lancet 20 is re-installed in the main body 30.
As a result, in addition to the puncture body 23 being forcibly held within the casing 22 during removal from the main body 30 after puncturing, when a used lancet 20 is installed to the main body 30, movement to a shot-ready completion state of the lancet device 10 is inhibited. This means that the danger of infection etc. with infectious diseases as a result of re-puncturing using a used lancet 20 at the side of the lancet device 10 is reliably avoided and it is possible to provide a very safe lancet device 10.

### <Features of Lancet Device 10>

### (1)

The lancet device 10 of the embodiment can also use a combination of two structures as a shot prevention mechanism for preventing re-shooting using a used lancet 20. In a first structure, as shown in Fig. 4 and Fig. 7(a), the positions for holding the puncture body 23 within the casing 22 are different before and after use. In a second structure, as shown in Fig. 7(a) to Fig. 8(b), the position for holding the puncture body 23 within the casing 22 being different for before and after use is utilized. When a used lancet 20 is installed in the main body 30, movement to the shot-ready completion state is inhibited as a result of the stopper plate 38 not acting and the setting release button 37 remaining in a pushed down state and not being pushed up.
As a result, movement to the shot-ready completion state does not be performed even when a used lancet 20 is erroneously installed in the main body 30. It is therefore not possible for the tip of the puncture needle 21 to protrude and for puncturing to be performed.
As a result, it is possible to reliably prevent the danger of a user becoming infected with an infectious disease via fluid etc. affixed to the tip of the puncture needle 21 as a result of performing puncturing using a used lancet 20.

### (2)

In the lancet device 10 of the embodiment, as shown in Fig. 4, a fitting portion composed of the groove 22c and the pinching portion 22b which are formed at the inner wall surface of the casing 22, and the groove 23c and the protruding portion 24a which are formed respectively at the puncture body 23 and the cap 24 is adopted as a mechanism for changing the holding position of the puncture body 23 within the casing 22 before and after use.
As a result, before puncturing, the protruding portion 24a of the cap 24 formed integrally with the puncture body 23 is held in the groove 22c formed towards the tip of the casing 22. On the other hand, a portion of the groove 23c of the puncture body 23 can be forcibly held by the pinching portion 22b of the casing 22 in the lancet 20 removed from the main body 30 after puncturing. As a result, it is possible to easily change the holding position of the puncture body 23 within the casing 22 before and after use.
Further, in the lancet 20 after use, the puncture body 23 is forcibly held within the casing 22, thereby the danger of the puncture needle 21 protruding from the end of the casing 22 is avoided after removal of the lancet 20.

### (3)

In the lancet device 10 of the embodiment, when a used lancet 20 is fitted to the main body 30 as shown in Fig. 8(a) and Fig. 8(b), movement of the lancet holder 32 is regulated by the rib 35c (refer to Fig. 9) formed at the inner wall surface of the housing 35.
As a result, contact position between a part of the lancet holder 32 and the rib 35c is set as a movement limited and the movement of the lancet 20 to the rear is regulated. It is therefore possible to prevent the setting release button 37 from being pushed upwards via the stopper plate 38 so as to move to a shot-ready completion state. As a result, with a simple structure, it is possible to prevent re-use of the lancet 20 after use and it is possible to provide a very safe lancet device 10.

### (4)

In the lancet device 10 of the embodiment, movement to the shot-ready completion state is performed by pushing the setting release button 37 upwards, as shown in Fig. 6(b).
It is therefore possible to reliably prevent the used lancet 20 from being used for puncturing again because it is not possible to push the setting release button 37 upwards in order to move to a shot-ready completion state when a used lancet 20 is installed.

### (5)

In the lancet device 10 of this embodiment, the stopper plate 38 is mainly used as the shot-ready movement portion for moving the structure in the main body 30 with the lancet 20 fitted within to the shot-ready completion state.
As a result, when the lancet 20 before use is installed, the rear end portion of the casing 22 of the lancet 20 moves the stopper plate 38 so as to release the latch portion 32c of the lancet holder 32. It is then possible for the setting release button 37 to be pushed up. On the other hand, when the lancet 20 is installed after use, movement of the lancet holder in the puncture direction is regulated as a result of a part of the lancet holder 32 abutting against the rib 35c formed at the inner wall surface of the housing 35 prior to the rear end of the casing 22 of the lancet 20 abutting with the stopper plate 38. The stopper plate 38 is therefore made to function and it is possible to inhibit pushing up of the setting release button 37. As a result, it is possible to reliably prevent re-puncturing using a used lancet 20 and provide a very safe lancet device 10 that is capable of.

### (ALTERNATIVE EMBODIMENTS)

An embodiment of the present invention has been explained above. However, the present invention is not limited to the above described embodiment, and a variety of changes may be made without departing from the scope of the invention.

### (A)

In the above embodiment, a description is given of an example taken as a means for inhibiting reuse of a used lancet 20 where a mechanism is adopted where it is not possible to release the latch portion 32c by the stopper plate 38 when a used lancet 20 is fitted. The setting release button 37 therefore cannot be pushed up and re-puncturing cannot be implemented. However, the present invention is not limited to this configuration.
In addition to re-puncturing being inhibited by it not being possible to push up the setting release button 37, it is also possible to provide a mechanism that inhibits reuse of a used lancet 20 by ensuring that, for example, it is not possible to insert a used lancet 20 as far as an appropriate position with respect to the main body 30.

### (B)

In the above embodiment, an explanation is given where the position of the puncture body 23 with respect to the casing 22 while removed from the main body 30 after puncturing is further to the rear side in a puncture direction than the position of the puncture body 23 with respect to the casing 22 while installed in the main body 30 before puncturing.
However, the present invention is not limited to this configuration.
For example, conversely to the above embodiment, it is also possible for the puncture body 23 to be held within the casing 22 after puncturing in such a manner that the position of the puncture body 23 with respect to the casing 22 while removed from the main body 30 after puncturing is further to the front side in a puncture direction than the position of the puncture body 23 with respect to the casing 22 while installed at the main body 30 before puncturing.
In this case, it is also possible to obtain the same results as described above by changing the position of the puncture body 23 with respect to the casing 22 before and after puncturing so as to ensure that the setting release button 37 cannot be pushed up when the side of the main body 30 is installed.

### (C)

In the above embodiment, an explanation is given taking the example of the lancet device 10 that is not provided with a re-cocking lever for carrying out re-cocking. However, the present invention is not limited to this configuration.
For example, it is also possible to provide a re-cocking lever as with typical lancet devices.
The configuration where a re-cocking lever is omitted such as in the above embodiment is preferable from the point of view of providing a highly safe lancet device that avoids the danger of carrying out re-cocking of a lancet once again and performing puncturing with a lancet that has been left in after puncturing.

### (D)

In the above embodiment, in addition to the setting release button 37 not being moved to the shot-ready completion state when a used lancet 20 is installed, a description is given of an example for avoiding the danger of reuse of a used lancet 20 by forcibly holding the puncture body 23 within the casing 22 in the lancet 20 removed from the main body 30 after puncturing. However, the present invention is not limited to this configuration.
For example, the forcible holding of the puncture body 23 within the casing 22 of the latter stage is not an essential aspect of the configuration and can also simply be changed to a holding position of the puncture body 23 with respect to the casing 22 before and after use.
However, from the point of view of improving safety during removal from the main body 30, as shown in the above embodiment, in addition to changing the holding position, it is also preferable to forcibly hold the puncture body 23 within the casing 22.

### (E)

In the above embodiment, an explanation is given taking as an example a mechanism constituted mainly by the lancet holder 32, the rib 35c formed at the inner wall surface of the housing 35, and the stopper plate 38 etc. as the mechanism for inhibiting movement of the lancet 20 after use to the shot-ready completion state when the lancet 20 is installed after use. However, the present invention is not limited to this configuration.
For example, it is possible to form a rib regulating movement of the lancet holder 32 at another member other than the inner wall surface of the housing 35 or at a position more to the front end side at the inner wall surface of the housing 35.
It is also possible for regulation of the movement of the stopper plate 38 in order to push up the setting release button 37 to be achieved by regulating the movement of the stopper plate 38 itself rather than by regulating movement of the lancet holder 32.

### INDUSTRIAL APPLICABILITY

The lancet device of the present invention is capable of ensuring a high degree of safety after use with a simple structure and is therefore widely applicable to puncture apparatus where a puncture needle protrudes in a predetermined direction and puncturing is carried out.

## Claims

1. A lancet device comprising:
a lancet having a puncture body with a puncture needle at a tip, and a lancet case housing the puncture body within so as to be movable in a puncture direction, with a holding position for the puncture body within the lancet case before puncturing and a holding position for the puncture body within the lancet case after removal once after puncturing are different;
a main body that has an opening portion the lancet is inserted into, and a lancet holder for holding the lancet inserted from the opening portion;
an biasing member provided within the main body, that provides a biasing force for causing the puncture needle to protrude towards the front end side with the whole of the lancet holder; and
a shot-ready movement portion provided within the main body, that enable the lancet to be moved to a shot-ready state by the biasing member only when the lancet is installed before puncturing.

2. The lancet device according to claim 1, the lancet comprising:
the puncture body that has a linking portion formed at an end portion on an opposite side to the puncture needle linking with the main body;
the lancet case that has a tubular portion housing the puncture body in a state where forward and reverse movement is possible in the puncture direction, and an opening formed at an end portion of the protruding side of the puncture needle; and
a fitting portion that holds the puncture body within the lancet case in a state where forward and reverse movement in the puncture direction is not possible when the linking portion of the puncture body is distanced from of the main body.

3. The lancet device according to claims 1 or 2, wherein the shot-ready movement portion moves the lancet to a shot-ready state in accordance with movement of the lancet holder in the puncture direction; and
the main body further comprises a movement regulating portion for regulating movement in the puncture direction of the lancet holder holding the lancet while the lancet is installed after puncturing.

4. The lancet device according to any one of claims 1 to 3, further comprising a shot button pressed when shooting the lancet, and
the shot-ready movement portion pushes the shot button that is normally pressed down upwards to a shot-ready state when the lancet is installed at the main body before puncturing.

5. The lancet device according to claim 4, wherein the shot-ready movement portion comprises a stopper plate that presses the shot button upwards so as to move towards the shot-ready state in accordance with movement of the lancet case in the puncture direction while the lancet is installed.
